# EUROPÄISCHE PATENTSCHRIFT

(11) **EP 2 915 876 B1**
(45) Veröffentlichungstag und Bekanntmachung des Hinweises auf die Patenterteilung: **30.11.2016**
(21) Anmeldenummer: 15155394.8
(22) Anmeldetag: 17.02.2015
(51) Int. Cl.: C12M 1/107, C12M 1/02, F28F 19/04, F28F 21/06

(54) **Wärmeübertragungsrohr für einen Biogasanlagenfermenter und Biogasanlagenfermenter**
Heat transfer tube for a biogas plant fermenter and biogas plant fermenter
Tuyau de transfert thermique pour un digesteur d'installation de biogaz et digesteur d'installation de biogaz

(30) Priorität: 05.03.2014 DE 202014100986 U
(43) Veröffentlichungstag der Anmeldung: 09.09.2015
(73) Patentinhaber: Uponor Innovation AB, 73061 Virsbo (SE)
(72) Erfinder: Roseen, Patrick, 72335 Västerås (SE); Bylin, Erika, 77635 Hedemora (SE)
(74) Vertreter: Epping - Hermann - Fischer

(56) Entgegenhaltungen:
- EP-A1- 0 814 126
- EP-A2- 1 728 824
- WO-A1-2011/128237
- DE-U1-202007 008 880
- "Introduction of EVAL EVOH for Geosynthetics & value in select applications", , 1. Mai 2010 (2010-05-01), XP055198359, Gefunden im Internet: URL:http://www.nyfederation.org/pdf2010/03 _armstrongr.pdf [gefunden am 2015-06-25]

## Beschreibung

Die Erfindung bezieht sich auf ein Wärmeübertragungsrohr für einen Biogasanlagenfermenter zur Übertragung von Wärme zwischen einem in dem Wärmeübertragungsrohr strömenden Medium und einem Substrat, welches das Wärmeübertragungsrohr zumindest teilweise umschließt. Die Erfindung bezieht sich ferner auf einen Biogasanlagenfermenter mit solch einem Wärmeübertragungsrohr.

Biogasanlagen mit einem Fermenter sind aus dem Stand der Technik bekannt. Diese Biogasanlagen umfassen üblicherweise einen Fermenter, in dem eine Fermentation eines Substrats erfolgt. Während einer solchen Fermentation wird das Substrat, bei dem es sich gewöhnlich um ein biologisch abbaubares (biodegradables) Material handelt, in mehreren Prozessen von Mikroorganismen abgebaut. Üblicherweise erfolgt die Fermentation ohne Sauerstoff und wird daher auch als anaerobe Gärung bezeichnet. Während der Fermentation wird aus Methan, Kohlendioxid und anderen Gasen bestehendes Biogas als eine Quelle erneuerbarer Energie erzeugt.

Damit die chemischen Prozesse während der Fermentation ordnungsgemäß ablaufen, muss ein Betriebstemperaturniveau aufrechterhalten werden. Beispielsweise erfolgt eine mesophile Gärung am besten bei ungefähr 32 bis 38 °C. Eine thermophile Gärung erfolgt beispielsweise am besten bei ungefähr 49 bis 57 °C oder sogar höheren Temperaturen.

Um ein solches Betriebstemperaturniveau während der Fermentation aufrechtzuerhalten, umfasst der Biogasanlagenfermenter eines oder mehrere Wärmeübertragungsrohre, in denen ein Medium strömt, um Wärme an das Substrat zu übertragen, welches eines oder mehrere Wärmeübertragungsrohre zumindest teilweise umschließt.

DE 202007008880 U1 betrifft ein Wärmeübertragungsrohr für einen Biogasfermenter und offenbart ein mehrschichtiges Kunststoffrohr umfassend ein Diffusionsschicht aus Sperrschichtkunststoffen, vorzugsweise EVOH oder PA.

Über ihre Lebensdauer werden diese Wärmeübertragungsrohre in Biogasanlagen normalerweise durch Metallcarbonatpräzipitate oder Sulfidpablagerungen (auch als Präzipitate bezeichnet) verstopft. Diese Ablagerungen verringern drastisch die Funktionsfähigkeit der Wärmeübertragungsrohre und/oder des Fermenters. Um ein solches Verstopfen zu vermeiden, werden beispielsweise dem in den Wärmeübertragungsrohren strömenden Medium chemische Konzentrate zugesetzt. Solche Konzentrate werden üblicherweise dazu verwendet, eine Korrosion von Metallteilen des Wärmeübertragungsrohrsystems wie Rohrleitungsfittings, welche diese Rohre verbinden, zu reduzieren. Des Weiteren wird ein derartiges Konzentrat verwendet, um zumindest teilweise Präzipitate bzw. Ablagerungen in den Rohren und Rohrkomponenten zu vermeiden. Eine andere Möglichkeit zum Verringern der Verstopfung in den Rohren ist die Verwendung von Filterelementen. Diese Filterelemente müssen selbstverständlich von Hand gereinigt und/oder ausgewechselt werden.

Eine Aufgabe der Erfindung besteht darin, ein Wärmeübertragungsrohr für einen Biogasanlagenfermenter und auch einen Fermenter für eine Biogasanlage zu offenbaren, die eine zuverlässige und präzise Wärmeübertragung begünstigen. Die Aufgabe wird durch die Merkmale der unabhängigen Ansprüche gelöst. Vorteilhafte Ausgestaltungen der Erfindung sind in den Unteransprüchen wiedergegeben.

Die Erfindung beruht auf dem Gedanken, die Permeation von Molekülen wie CO₂ und H₂S in das Medium innerhalb eines Wärmeübertragungsrohrs zu verringern. Diese Moleküle diffundieren von dem umgebenden Substrat in dem Fermenter in das Medium im Rohr. Eine hohe Diffusion oder eine hohe Diffusionsrate solcher Moleküle hat die Wirkung, dass der pH-Wert des Mediums in den Wärmeübertragungsrohren sinkt.

Ein niedrigerer pH-Wert erhöht die Azidität des Mediums und somit nimmt die Korrosivität zu, die zu Metallablagerungen in dem Wärmeübertragungsrohr führt. Durch das vorgeschlagene Konzept werden die Diffusionseigenschaften des Wärmeübertragungsrohrs so verbessert, dass viel weniger, nur wenige oder praktisch keine Moleküle wie CO₂- und H₂S-Moleküle in das Medium des Rohrs diffundieren. Dies bewirkt, dass die Korrosion des Wärmeübertragungsrohrs oder von Metallkomponenten wie Fittings stark verringert wird und das Verstopfen im Vergleich zu herkömmlichen Rohren, die eine sehr viel höhere Diffusion zulassen, minimiert wird.

Gemäß einem ersten Aspekt der Erfindung wird ein Wärmeübertragungsrohr für einen Biogasanlagenfermenter zur Übertragung von Wärme zwischen einem in dem Wärmeübertragungsrohr strömenden Medium und einem das Wärmeübertragungsrohr zumindest teilweise umschließenden Substrat offenbart. Das Wärmeübertragungsrohr umfasst eine innere Schicht für den Kontakt mit dem Medium und eine Diffusionssperrschicht zum Verhindern einer Permeation von Molekülen des Substrats in das Medium. Die Diffusionssperrschicht besteht aus Ethylen-Vinylalkohol, EVOH, und ein Gehalt an Ethylen der Diffusionssperrschicht beträgt mindestens mehr als 32%.

Durch Ändern der Zusammensetzung der Diffusionssperrschicht zu einem Grad mit einem höheren Ethylengehalt, der mindestens mehr als 32% beträgt, wird die Diffusion von chemischen Molekülen in das Medium im Wärmeübertragungsrohr überraschenderweise im Vergleich zu herkömmlichen Heizungsrohren mit einer gleichen Wanddicke einer Diffusionssperrschicht für sehr feuchte Umgebungsbedingungen, bei denen eine relative Feuchte mindestens mehr als 90%, insbesondere 100%, beträgt, erheblich verringert. Derartige feuchte Bedingungen herrschen beispielsweise in Biogasanlagenfermentern vor. Je höher der Ethylengehalt ist, umso höher ist im Allgemeinen eine Diffusionsrate der Moleküle und umso größer ist der Widerstand gegen die Aufnahme von Feuchtigkeit. Bei steigendem Ethylengehalt nimmt die Diffusionssperrschicht daher bei derartigen feuchten Bedingungen weniger Feuchtigkeit auf, so dass eine Diffusion der Moleküle verhindert wird und die Diffusionsrate der Moleküle verringert wird. Die Diffusionssperrschicht bleibt, mit anderen Worten, durch die Einwirkung von Feuchtigkeit intakt. Folglich wird der pH-Wert des Mediums durch die Permeation von Molekülen in das Medium nicht wesentlich gesenkt und eine zur Ablagerung von Metallcarbonaten oder Sulfiden führende Korrosion, insbesondere von Metallteilen wie den vorgenannten Fittings, Pumpen oder Wärmeaustauschern, wird verringert oder vermieden. Daher wird die Verstopfung des Wärmeübertragungsrohrs verringert. Ein derartiger Ethylengehalt garantiert ferner eine sichere und gefahrlose Verwendung des Wärmeübertragungsrohrs bei hohen Betriebstemperaturen.

Gegenüber herkömmlichen Rohren ermöglicht der Ethylengehalt eine sehr viel dünnere Diffusionssperrschicht, um eine im Vergleich zu herkömmlichen Rohren gleiche Diffusionssperre zu erhalten. Als zusätzlicher Vorteil kommt hinzu, dass keine chemischen Konzentrate erforderlich sind, um das Verstopfen oder das Absinken des pH-Werts zu vermeiden. Ferner sind keine zusätzlichen Bauteile wie Filterelemente oder dergleichen erforderlich, was, unter anderem, die Gesamtkosten des Wärmeübertragungsrohrs oder Systems und seines Biogasanlagenfermenters senkt.

Es sei an dieser Stelle bemerkt, dass die Diffusion von Molekülen auf eine gegebene Zeitspanne bezogen ist. Das heißt, dass durch die Erhöhung des Ethylengehalts der Diffusionssperrschicht eine Diffusionsrate der in das Medium permeierenden Moleküle verringert wird.

Bei verschiedenen vorteilhaften Ausgestaltungen der Erfindung beträgt der Ethylengehalt mindestens mehr als 34%, 36%, 38%, 40%, 42% oder 44%. Im Allgemeinen beträgt ein optimaler Ethylengehalt zwischen 40% und 50% bei einer relativen Feuchte von 100%. Insbesondere stellt ein Gehalt von 44% eine sehr hohe Diffusionssperre gegen die Moleküle bei einer relativen Feuchte von 100% bereit.

Bei verschiedenen vorteilhaften Ausgestaltungen beträgt eine Wanddicke der Diffusionssperrschicht zwischen 0,04 mm und 1,5 mm. Vorzugsweise beträgt die Wanddicke ungefähr 200 µm.

Grundsätzlich ist eine Diffusion durch die Diffusionssperrschicht proportional zu einer Wanddicke der Diffusionssperrschicht, insbesondere linear proportional. Zusammen mit dem vorgenannten Ethylengehalt der Diffusionssperrschicht begünstigt eine solche Wanddicke der Diffusionssperrschicht, dass weniger, nur wenige oder praktisch gar keine Moleküle in das Medium des Wärmeübertragungsrohrs permeieren.

Bei verschiedenen vorteilhaften Ausgestaltungen ist die Wanddicke der Diffusionssperrschicht und/oder des Wärmeübertragungsrohrs so angepasst, dass ein Gesamtaußendurchmesser des Wärmeübertragungsrohrs zu entsprechenden Rohrleitungsfittings oder anderen Systemkomponenten wie T-Stücken oder dergleichen passt. Insbesondere müssen der Gesamtaußendurchmesser des Wärmeübertragungsrohrs und ein Innendurchmesser, der durch die Innenschicht des Wärmeübertragungsrohrs definiert wird, so angepasst sein, dass sich das Wärmeübertragungsrohr für eine Schnellverbindungstechnik wie die Q&E ("Quick and Easy") Verbindungstechnik der Firma Uponor eignet. Bei der Q&E-Technik wird ein Ring, insbesondere ein Kunststoffring, an einem Ende auf das Wärmeübertragungsrohr geschoben, wobei der Außendurchmesser des Wärmeübertragungsrohrs nicht einen vorbestimmten Wert überschreitet. Durch Ändern des Ethylengehalts der Diffusionssperrschicht eignet sich das Wärmeübertragungsrohr für die Q&E-Technik, ohne die Wanddicke weiter zu erhöhen, und verhindert die Permeation von Molekülen, wie oben erwähnt. Beispielsweise werden Rohre mit einem Außendurchmesser von ungefähr 20 mm oder zwischen 19,5 mm und 20,3 mm verwendet.

Bei verschiedenen vorteilhaften Ausgestaltungen umfasst das Wärmeübertragungsrohr ferner eine äußere Schutzschicht und eine oder mehrere Haftschichten, welche die Schichten, insbesondere die Innenschicht, die Diffusionssperrschicht und die äußere Schutzschicht, miteinander verbinden. Beispielsweise ist zwischen der Innenschicht und der Diffusionssperrschicht eine Haftschicht angeordnet. Durch Hinzufügen einer zusätzlichen äußeren Schutzschicht kann das Wärmeübertragungsrohr Temperaturen bis zu beispielsweise 90 °C ausgesetzt werden. Ferner können die Wärmebeständigkeit und die chemische Beständigkeit des Rohrs erhöht werden. Beispielsweise kann die Haftschicht lineares Polyethylen niederer Dichte (PE-LLD) umfassen.

Bei verschiedenen Ausführungsformen ist das Wärmeübertragungsrohr geeignet, mit einem Rohrleitungsfitting durch eine Schnellverbindungstechnik, insbesondere die so genannte Quick & Easy Verbindungstechnik von der Firma Uponor, verbunden zu werden.

Das oben erwähnte Substrat umfasst mindestens einen der folgenden biologisch abbaubaren Abfallstoffe:
- Gülle;
- Altpapier;
- Rasenschnitt;
- Essensreste;
- Abwasser;
- Energiepflanzen; oder
- tierische Abfälle.

Die Diffusionssperrschicht hindert mindestens eines der folgenden Moleküle am Permeieren in das Medium: Kohlenstoffdioxid oder Schwefelwasserstoff.

Gemäß einem zweiten Aspekt der Erfindung wird ein Biogasanlagenfermenter offenbart, der ein Wärmeübertragungsrohr gemäß dem ersten Aspekt der Erfindung und ein Substrat umfasst, welches das Wärmeübertragungsrohr zumindest teilweise umschließt.

Der Fermenter begünstigt im Wesentlichen die vorgenannten Vorteile und Funktionen.

Eine beispielhafte Ausführungsform der Erfindung wird im Folgenden mit Hilfe von schematischen Zeichnungen und Bezugsziffern erläutert. Gleiche Bezugsziffern bezeichnen Elemente oder Bauteile mit gleichen Funktionen. Insofern Elemente oder Bauteile einander in der Funktion entsprechen, wird ihre Beschreibung nicht in jeder der folgenden Figuren wiederholt.

Die Figuren sind wie folgt:
- Figur 1: ist eine schematische Schnittansicht eines Wärmeübertragungsrohrs gemäß einer Ausführungsform der Erfindung, und
- Figur 2: ist eine schematische Teilansicht eines Biogasanlagenfermenters.

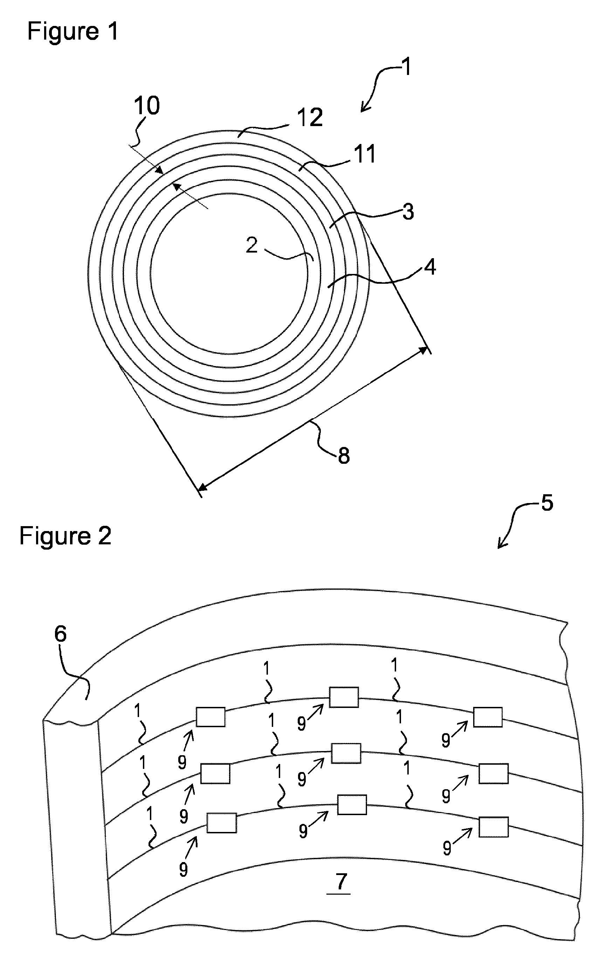

Figur 1 zeigt schematisch eine Schnittansicht eines Wärmeübertragungsrohrs 1 für einen Biogasanlagenfermenter 5, der teilweise, dreidimensional und schematisch in Figur 2 dargestellt ist. Das Wärmeübertragungsrohr 1 ist ausgestaltet, um Wärme zwischen einem in dem Wärmeübertragungsrohr 1 strömenden Medium und einem Substrat 7 des Biogasanlagenfermenters zu übertragen, welches das Wärmeübertragungsrohr 1 zumindest teilweise umschließt (das Substrat sowie dessen Umschließen ist in Figur 2 nicht dargestellt).

Das Wärmeübertragungsrohr 1, welches ein mehrschichtiges Rohr ist, umfasst eine Innenschicht 2 für den Kontakt mit dem Medium. Das durch das Wärmeübertragungsrohr 1 strömende Medium ist üblicherweise Wasser, kann aber auch jedes andere zur Wärmeübertragung geeignete Fluid sein.

Die Innenschicht 2 umfasst vernetztes Polyethylen (PE-X), das eine hohe Säurebeständigkeit, eine hohe Wärmebeständigkeit und/oder eine hohe Druckbeständigkeit ermöglicht. Alternativ kann die Innenschicht Polypropylen (PP), Polybutylen (PB), Polyethylen niederer Dichte (PE-LD) oder Polyethylen mit erhöhter Temperaturbeständigkeit (PE-RT) umfassen.

Das Wärmeübertragungsrohr 1 umfasst ferner eine die Innenschicht 2 umgebende Diffusionssperrschicht 3. Die Diffusionssperrschicht 3 ist mit der Innenschicht 2 durch eine Haftschicht 4 verbunden, die zwischen der Innenschicht 2 und der Diffusionssperrschicht 3 angeordnet ist. Die Diffusionssperrschicht 3 besteht aus Ethylen-Vinylalkohol (EVOH). Dabei beträgt ein Ethylengehalt der Diffusionssperrschicht 3 44%. Die Haftschicht 4 umfasst PE-LLD.

Außerdem umfasst das Wärmeübertragungsrohr 1 eine weitere optionale Haftschicht 11, welche die Diffusionssperrschicht 3 umgibt, und eine äußere Schutzschicht 12, welche die weitere Haftschicht 11 umgibt. Die weitere Haftschicht 11 kann analog zur Haftschicht 4 sein, sie kann aber auch aus einem anderen Material bestehen. Die äußere Schutzschicht 12 besteht aus Polyethylen niederer Dichte (PE-LD). PE-LD ermöglicht eine hohe Wärme- oder Temperaturbeständigkeit.

Das Wärmeübertragungsrohr 1 ist ausgebildet, um in den Biogasanlagenfermenter 5 gemäß Figur 2 eingebaut zu werden und um das Substrat 7 im Fermenter 5 zu erwärmen. Das Erwärmen ermöglicht den ordnungsgemäßen Ablauf der Gärungs- oder Fermentationsprozesse des Substrats 7, wie oben erläutert wurde. Der Fermenter 5 ist beispielhaft in Figur 2 in einer perspektivischen Schnittansicht dargestellt. Es sei an dieser Stelle bemerkt, dass der Fermenter 5 jede beliebige Ausführungsform haben kann, wobei nur ein Wärmeübertragungsrohr 1 installiert ist, um Wärme auf das oder von dem Substrat 7 in einem solchen Fermenter 5 zu übertragen. Das Substrat 7 umfasst viel Wasser, so dass eine gesamte Umgebung innerhalb des Fermenters 5 feucht ist. Das heißt, dass eine relative Feuchte im Fermenter 100% beträgt. Daher ist das Wärmeübertragungsrohr 1 sehr feuchten Umgebungsbedingungen ausgesetzt.

Der Fermenter 5 umfasst eine Seitenwand 6. Im Fermenter 5 sind mehrere Wärmeübertragungsrohre 1 mit Hilfe von Befestigungselementen (nicht dargestellt) an der Seitenwand 6 befestigt. Des Weiteren sind die Wärmeübertragungsrohre 1 durch Rohrleitungsfittings 9, die in Figur 2 schematisch dargestellt sind, miteinander verbunden. Die Rohrleitungsfittings 9 bestehen aus Kunststoff. Alternativ können keine Fittings oder andere Arten von Fittings oder andere Rohrverbindungselemente verwendet werden. Beispielsweise kann der Fermenter 5 Verteiler in der Seitenwand 6 umfassen, die mit mehreren Wärmeübertragungsrohren 1 verbunden sind. Dabei können die Verteiler oder ihre Anschlüsse aus Metall wie nichtrostendem Stahl bestehen.

Des Weiteren ist das Substrat 7, das im Fermenter 5 bereitgestellt wird, biologisch abbaubar bzw. biodegradabel. Das Substrat umfasst mindestens eines von Folgendem: Gülle, Altpapier, Rasenschnitt, Essensreste, Abwasser, Energiepflanzen und/oder tierische Abfälle.

Wie oben erwähnt, muss eine Betriebstemperatur stabil sein und aufrechterhalten werden, damit die Gärungsprozesse beziehungsweise die Fermentation des Substrats 7 ordnungsgemäß ablaufen. Daher übertragen die Wärmeübertragungsrohre 1, die durch Pressfittings 9 oder dergleichen angeschlossen sind, Wärme an das Substrat 7. Die Wärmeübertragungsrohre 1 sind zumindest teilweise mit dem Substrat 7 umgeben und/oder in Kontakt.

Während der Fermentation des Substrats 7 werden Moleküle wie CO₂ und H₂S oder andere Moleküle und chemische Stoffe erzeugt, die aus dem umgebenden Substrat 7 durch die Wärmeübertragungsrohre 1 in das Wasser in den Wärmeübertragungsrohren 1 diffundieren. Aufgrund des hohen Gehalts des Ethylens in der Diffusionssperrschicht 3 des Wärmeübertragungsrohrs 1 unter den sehr feuchten Bedingungen, ist eine solche Diffusion erheblich herabgesetzt und/oder wird vermieden. Da, wie zu Anfang erwähnt, weniger Feuchtigkeit von der Diffusionssperrschicht 3 aufgenommen wird und somit eine geringere Diffusion erfolgt, sinkt der pH-Wert des Wassers in den Wärmeübertragungsrohren 1 nicht, was zu einer höheren Azidität des Wassers im Wärmeübertragungsrohr 1 führen würde. Durch einen niedrigen pH-Wert würde eine Korrosion von Metallteilen der Wärmeübertragungsrohre 1, des Biogasfermenters 5, der Fittings 9, der Pumpen, der Wärmeaustauscher oder dergleichen bewirkt. Die Korrosion würde zu Ablagerungen von bspw. Metallcarbonaten oder Sulfiden führen, welche die Wärmeübertragungsrohre 1 verstopfen und die Funktionsfähigkeit drastisch herabsetzen. Daher wird eine verbesserte Lebensdauer des Biogasfermenters 5 und/oder der Wärmeübertragungsrohre 1 und/oder anderer Metallteile erzielt.

Gemäß der dargestellten Ausführungsform der Erfindung umfasst die Diffusionssperrschicht 3 eine Wanddicke 10 von 200 µm. Im Allgemeinen ist die Diffusion durch die Diffusionssperrschicht proportional zur Wanddicke 10 der Schicht 3. Bei der dargestellten Ausführungsform ist die Wanddicke 10 der Diffusionssperrschicht 3 so dick wie möglich, wobei ein Außendurchmesser 8 des Wärmeübertragungsrohrs 1 einen vorbestimmten Wert nicht überschreitet. Ein solcher vorbestimmter Wert kann beispielsweise ein Rohrdurchmesser von 20 mm sein. Eine dickere Diffusionssperrschicht 3 verbessert ferner die Verhinderung der Permeation der Moleküle des Substrats in das Medium.

Alternativ ist die Wanddicke 10 auch größer, beispielsweise mehr als 1,0 mm, wodurch die Diffusionssperreigenschaften des Rohrs 1 weiter verbessert werden.

Durch die Kombination der Wanddicke 10 mit dem Ethylengehalt der Diffusionssperrschicht 3 wird die Permeation der Moleküle durch das Wärmeübertragungsrohr 1 in das Wasser stark verringert. Mit anderen Worten weist das Wärmeübertragungsrohr 1 im Vergleich zu einem herkömmlichen Rohr mit einem sehr viel niedrigeren Ethylengehalt und mit der gleichen Wanddicke wie das Wärmeübertragungsrohr 1 eine überraschend bessere Diffusionssperre unter sehr feuchten Bedingungen auf. Um eine gleiche Diffusionssperre wie bei herkömmlichen Rohren zu erhalten, kann des Weiteren die Wanddicke des Wärmeübertragungsrohrs 1 unter derart feuchten Bedingungen sehr viel kleiner sein, wodurch Material und Kosten gespart werden.

Als ein Beispiel werden nachstehend ein herkömmliches Rohr und das Wärmeübertragungsrohr 1 gemäß der mit Hilfe der Figuren 1 und 2 beschriebenen Ausführungsform verglichen. Beide Rohre sind den gleichen sehr feuchten Bedingungen bei 20 °C ausgesetzt. Beide Rohre umfassen einen gleichen Aufbau, wobei das herkömmliche Rohr eine Diffusionssperrschicht mit einem Ethylengehalt von 32% umfasst und das Wärmeübertragungsrohr 1 einen Ethylengehalt von 44% umfasst. Eine Diffusionsrate von CO₂ durch die Diffusionssperrschicht des herkömmlichen Rohrs beträgt 220 und eine Diffusionsrate durch die Diffusionssperrschicht 3 des Wärmeübertragungsrohrs 1 beträgt 128. Folglich ist die Diffusion sehr viel geringer, so dass der pH-Wert des Wassers im Wärmeübertragungsrohr 1 nicht sehr stark gesenkt wird.

Des Weiteren ist das Wärmeübertragungsrohr 1 wahlweise geeignet, mit einem Rohrleitungsfitting durch eine Schnellverbindungstechnik, insbesondere durch die Quick & Easy Verbindungstechnik von der Firma Uponor, verbunden zu werden. Um die vorgenannten Diffusionseigenschaften unter sehr feuchten Bedingungen bereitzustellen, überschreitet der Außendurchmesser 8 des Wärmeübertragungsrohrs 1, wie oben erwähnt, aufgrund des Ethylengehalts von 44% nicht einen vorbestimmten Wert. Somit kann der Außendurchmesser 8 so angepasst sein, dass ein Ring für die Q&E-Technik auf ein Ende des Wärmeübertragungsrohrs 1 geschoben werden kann.

Außerdem umfasst das Wärmeübertragungsrohr 1 aufgrund des beschriebenen Aufbaus des Wärmeübertragungsrohrs 1 eine höhere Temperaturbeständigkeit bis 90 °C oder auch darüber. In der dargestellten Ausführungsform gemäß Figur 1 ist die Diffusionssperrschicht 3 des Wärmeübertragungsrohrs 1 zwischen der äußeren Schutzschicht 12 und der Innenschicht 2 des Wärmeübertragungsrohrs 1 angeordnet. Die Diffusionssperrschicht 3 kann alternativ zwischen einer Außenseite und der Innenschicht 2 des Rohrs 1 angeordnet sein. Bei verschiedenen Ausführungsformen kann die Diffusionssperrschicht 3 auch die Innenschicht des Rohrs 1 bilden und somit zwischen dem Inneren und der Innenschicht 2 des Rohrs 1 angeordnet sein.

Bei verschiedenen Ausführungsformen kann das Wärmeübertragungsrohr 1 eine weitere Schicht umfassen, die zwischen der Innenschicht 2 und der Diffusionssperrschicht 3 oder zwischen der Diffusionssperrschicht 3 und einer Außenseite des Rohrs 1 angeordnet ist. Diese weitere Schicht kann aus den vorgenannten Kunststoffen entsprechend der Innenschicht 2 bestehen. Die weitere Schicht kann auch an der Diffusionssperrschicht 3 und/oder der Innenschicht 2 durch eine dazwischen angeordnete Haftschicht 4 oder 11 angebracht sein. Ein solches Wärmeübertragungsrohr 1 ist ferner gegen chemische Angriffe geschützt und/oder verbessert eine Wärmeübertragung.

Es sei an dieser Stelle bemerkt, dass ein Wärmeübertragungsrohr 1 gemäß der Erfindung im Allgemeinen die Diffusionssperrschicht 3, wie sie oben beschrieben wurde, umfassen muss, damit eine Diffusion von Molekülen in das Medium innerhalb des Wärmeübertragungsrohrs 1 verhindert und/oder verringert wird.

### Bezugszeichen:

- 1: Wärmeübertragungsrohr
- 2: Innenschicht
- 3: Diffusionssperrschicht
- 4: Haftschicht
- 5: Fermenter
- 6: Seitenwand
- 7: Substrat
- 8: Außendurchmesser
- 9: Fitting
- 10: Wanddicke
- 11: Haftschicht
- 12: äußere Schutzschicht

## Patentansprüche

1. Wärmeübertragungsrohr (1) für einen Biogasanlagenfermenter (5) zur Übertragung von Wärme zwischen einem in dem Wärmeübertragungsrohr (1) strömenden Medium und einem das Wärmeübertragungsrohr (1) zumindest teilweise umschließenden Substrat (7), umfassend eine Innenschicht (2) für den Kontakt mit dem Medium und eine Diffusionssperrschicht (3) zum Verhindern einer Permeation von Molekülen des Substrats (7) in das Medium, wobei
- die Diffusionssperrschicht (3) aus Ethylen-Vinylalkohol, EVOH, besteht, und
- ein Ethylengehalt mindestens mehr als 32% beträgt.

2. Wärmeübertragungsrohr (1) nach Anspruch 1, wobei der Ethylengehalt mindestens mehr als 36% beträgt.

3. Wärmeübertragungsrohr (1) nach Anspruch 1, wobei der Ethylengehalt mindestens 44% oder mehr beträgt.

4. Wärmeübertragungsrohr (1) nach einem der vorhergehenden Ansprüche, wobei eine Wanddicke (10) der Diffusionssperrschicht (3) zwischen 0,04 mm und 1,5 mm, insbesondere 0,2 mm, beträgt.

5. Wärmeübertragungsrohr (1) nach einem der vorhergehenden Ansprüche, wobei eine Haftschicht (4, 11) zwischen der Innenschicht (2) und der Diffusionssperrschicht (3) oder zwischen der Diffusionssperrschicht (3) und einer äußeren Schutzschicht (12) angeordnet ist.

6. Wärmeübertragungsrohr (1) nach einem der vorhergehenden Ansprüche, das ferner eine äußere Schutzschicht (12) umfasst, welche die Diffusionssperrschicht (3) und die Innenschicht (2) umgibt.

7. Wärmeübertragungsrohr (1) nach einem der vorhergehenden Ansprüche, wobei die Innenschicht (2) und/oder die äußere Schutzschicht (12) Polypropylen, PP, Polybutylen, PB, Polyethylen mit erhöhter Temperaturbeständigkeit, PE-RT, Polyethylen niederer Dichte, PE-LD, oder quervernetztes Polyethylen, PE-X, umfassen.

8. Wärmeübertragungsrohr (1) nach einem der vorhergehenden Ansprüche, das geeignet ist, durch eine Schnellverbindungstechnik mit einem Rohrleitungsfitting verbunden zu werden.

9. Wärmeübertragungsrohr (1) nach einem der vorhergehenden Ansprüche, wobei ein Außendurchmesser (8) des Wärmeübertragungsrohrs (1) zwischen 19,5 mm und 20,3 mm, insbesondere 20 mm, oder mehr beträgt.

10. Wärmeübertragungsrohr (1) nach einem der vorhergehenden Ansprüche, wobei die Diffusionssperrschicht (3) mindestens eines der folgenden Moleküle am Permeieren in das Medium hindert:
- Kohlenstoffdioxid, CO₂; oder
- Schwefelwasserstoff, H₂S.

11. Biogasanlagenfermenter (5), der ein Wärmeübertragungsrohr (1) nach einem der vorhergehenden Ansprüche und ein Substrat (7) umfasst, welches das Wärmeübertragungsrohr (1) zumindest teilweise umschließt.

12. Biogasanlagenfermenter (5) nach Anspruch 11, wobei das Substrat (7) mindestens einen der folgenden biologisch abbaubaren Abfallstoffe umfasst:
- Gülle;
- Altpapier;
- Rasenschnitt;
- Essensreste;
- Abwasser;
- Energiepflanzen; oder
- tierische Abfälle.

## Claims

1. A heat transfer pipe (1) for a biogas plant fermenter (5) to transfer heat between a medium flowing in the heat transfer pipe (1) and a substrate (7) at least partially enclosing the heat transfer pipe (1), comprising an inner layer (2) for contact with the medium and a diffusion barrier layer (3) to prevent a permeation of molecules of the substrate (7) into the medium, wherein
- the diffusion barrier layer (3) consists of ethylene vinyl alcohol, EVOH, and
- an ethylene content is at least more than 32%.

2. The heat transfer pipe (1) according to claim 1, wherein the ethylene content is at least more than 36%.

3. The heat transfer pipe (1) according to claim 1, where in the ethylene content is at least 44% or more.

4. The heat transfer pipe (1) according to one of the preceding claims, wherein a wall thickness (10) of the diffusion barrier layer (3) is between 0.04 mm and 1.5 mm, in particular 0.2 mm.

5. The heat transfer pipe (1) according to one of the preceding claims, wherein an adhesion layer (4, 11) is arranged between the inner layer (2) and the diffusion barrier layer (3) or between the diffusion barrier layer (3) and an outer protective layer (12).

6. The heat transfer pipe (1) according to one of the preceding claims, that further comprises an outer protective layer (12), which encloses the diffusion barrier layer (3) and the inner layer (2).

7. The heat transfer pipe (1) according to one of the preceding claims, wherein the inner layer (2) and/or the outer protective layer (12) comprises polypropylene, PP, polybutylene, PB, polyethylene with a higher temperature resistance, PE-RT, low-density polyethylene, PE-LD, or crosslinked polyethylene, PE-X.

8. The heat transfer pipe (1) according to one of the preceding claims, that is suitable to be connected with a pipeline fitting by a quick connection technology.

9. The heat transfer pipe (1) according to one of the preceding claims, wherein an outer diameter (8) of the heat transfer pipe (1) is between 19.5 mm and 20.3 mm, in particular 20 mm.

10. The heat transfer pipe (1) according to one of the preceding claims, wherein the diffusion barrier layer (3) prevents at least one of the following molecules from permeating the medium:
- carbon dioxide, CO₂; or
- hydrogen sulfide, H₂S.

11. A biogas plant fermenter (5), which comprises a heat transfer pipe (1) according to one of the preceding claims and a substrate (7), which at least partially encloses a heat transfer pipe (1).

12. The biogas plant fermenter (5) according to claim 11, wherein the substrate (7) comprises at least one of the following biodegradable waste materials:
- liquid manure;
- wastepaper;
- lawn cuttings;
- food wastes;
- wastewater;
- energy crops; or
- animal waste.

## Revendications

1. Un tube de transfert de chaleur (1) pour un fermenteur d'installation de biogaz (5) pour transférer de la chaleur entre un moyen qui circule dans le tube de transfert de chaleur (1) et un substrat (7) entourant au moins partiellement le tube de transfert de chaleur (1), comprenant une couche interne (2) pour le contact avec le moyen et une couche barrière de diffusion (3) pour éviter une infiltration de molécules du substrat (7) dans le moyen, dans lequel
- la couche barrière de diffusion (3) est composée d'éthylène alcool vinylique, EVOH, et
- une teneur d'éthylène est au minimum de plus de 32%.

2. Le tube de transfert de chaleur (1) selon la revendication 1, dans lequel la teneur d'éthylène est au minimum de plus de 36%.

3. Le tube de transfert de chaleur (1) selon la revendication 1, dans lequel la teneur d'éthylène est au minimum de 44% ou plus.

4. Le tube de transfert de chaleur (1) selon une des revendications précédentes, dans lequel une épaisseur de paroi (10) de la couche barrière de diffusion (3) se trouve entre 0,04 mm et 1,5 mm, notamment 0,2 mm.

5. Le tube de transfert de chaleur (1) selon une des revendications précédentes, dans lequel une couche adhésive (4, 11) est disposée entre la couche interne (2) et la couche barrière de diffusion (3) ou entre la couche barrière de diffusion (3) et une couche protectrice externe (12).

6. Le tube de transfert de chaleur (1) selon une des revendications précédentes, qui comprend en outre une couche protectrice externe (12) qui entoure la couche barrière de diffusion (3) et la couche interne (2).

7. Le tube de transfert de chaleur (1) selon une des revendications précédentes, dans lequel la couche interne (2) et/ou la couche protectrice externe (12) comprend du polypropylène, PP, du polybutylène, PB, du polyéthylène avec une meilleure résistance à la température, PE-RT, du polyéthylène à basse densité, PE-LD, ou du polyéthylène réticulé PE-X.

8. Le tube de transfert de chaleur (1) selon une des revendications précédentes, qui est convenable pour être relié à un raccord de canalisation grâce à une technologie de raccordement rapide.

9. Le tube de transfert de chaleur (1) selon une des revendications précédentes, dans lequel un diamètre externe (8) du tube de transfert de chaleur (1) se trouve entre 19,5 mm et 20,3 mm, notamment 20 mm.

10. Le tube de transfert de chaleur (1) selon une des revendications précédentes, dans lequel la couche barrière de diffusion (3) évite qu'au moins une des molécules suivantes s'infiltre dans le moyen:
- dioxyde de carbone, CO₂; ou
- sulfure d'hydrogène, H₂S.

11. Un fermenteur d'installation de biogaz (5), qui comprend un tube de transfert de chaleur (1), selon une des revendications précédentes, et un substrat (7), qui entoure au moins partiellement un tube de transfert de chaleur (1).

12. Le fermenteur d'installation de biogaz (5) selon la revendication 11, dans lequel le substrat (7) comprend au moins un des déchets biodégradables suivants:
- du purin ;
- des vieux papiers ;
- des tontes de gazon ;
- des déchets alimentaires ;
- des eaux usées ;
- des cultures énergétiques ; où
- des déjections animales.
